# EUROPÄISCHE PATENTANMELDUNG

(11) **EP 2 270 237 A2**
(43) Veröffentlichungstag der Anmeldung: **05.01.2011**
(21) Anmeldenummer: 10005740.5
(22) Anmeldetag: 02.06.2010
(51) Int. Cl.: C13K 1/02, C13K 13/00, C12N 9/28, A23K 1/00, A61K 31/716, C08B 30/04, C12N 9/34, C12P 19/04, C08B 37/00

(54) **Verfahren zur Herstellung von Arabinoxylan**

(30) Priorität: 02.06.2009 DE 102009023469
(71) Anmelder: Jäckering Mühlen- Nährmittelwerke Gmbh, 59067 Hamm (DE)
(72) Erfinder: Roick, Thomas Dr., 48317 Drensteinfurt (DE)
(74) Vertreter: Bausch, Thomas

(57) **Zusammenfassung**

Verfahren zur Herstellung von wasserlöslichem Arabinoxylan aus Weichweizenmehl mittels der Verfahrensschritte Herstellung einer Mehl-Wasser-Suspension, Einhalten einer Ruhezeit der Mehl-Wasser-Suspension von 3-10 Minuten, Verdünnung der Mehl-Wasser-Suspension mit Kreislaufwasser, Dekantieren der Mehl-Wasser-Suspension mittels eines Drei-Phasen-Dekanters in einem Zentrifugalfeld, Separation des erhaltenen Oberlaufs in einem weiteren Zentrifugalfeld, Rückführung der im weiteren Zentrifugalfeld erhaltenen schweren Fraktion in den Stärkegewinnungskreislauf, Zusetzung von α-Amylase zur im weiteren Zentrifugalfeld erhaltenen leichten Fraktion, Dextrinieren der leichten Fraktion mit einem Jet-Kocher, Lagerung des Hydrolysats mit Flokkulation und Sedimentation in einem Reaktionsbehälter über 0,3 bis 13 Stunden und einem pH-Wert von 1 bis 10, Abzentrifugieren des Sediments, Mahltrocknung des Sediments, Verzuckern des geklärten und dextrinierten Hydrolysates in einem Rührreaktor mittels Glucoamylase unter gleichzeitiger Proteolyse mit einer Mischung einer alkalischen und einer neutralen Protease, Ultrafiltration des Hydrolysates über eine mehrstufige Ultrafiltration, wobei ein Permeat und ein Retentat erhalten werden und das Retentat mengenmäßig nur noch höchstens ein Fünftel der Ausgangsmenge beträgt. Eindampfung des Permeats, bis eine Konzentration von 50 bis 90 % Trockensubstanz erreicht ist, Eindampfung des Retentats, bis eine Konzentration von 20 bis 50 % Trockensubstanz erreicht ist, Rückführung der beiden Verdampferbrüdenkondensate in den Stärkeprozess, Präzipitation des eingedampften Retentats bis zu einer Ethanolkonzentration von 60 bis 80 Massenprozent in einem Kreislauf, der in Semi-Batch-Fahrweise betrieben wird und gebildet wird durch einen Rührreaktor, enthaltend 94 bis 99 Volumenprozent Ethanol bei einer Temperatur von 5 bis 70 °C, weiterhin einen schnelllaufenden Rotor-Stator-Mischer, eine Zugabemöglichkeit von konzentriertem Retentat in einen Bypass-Einlass des Rotor-Stator-Mischers unter Vakuumbedingungen, Filtern und mindestens einmaligem Waschen des Filterkuchens mit 94 bis 99 Volumenprozent aufweisendem Ethanol sowie anschließender Mahltrocknung des auf die beschriebene Weise hergestellten wasserlöslichen Ballaststoffkonzeritrats, und Aufarbeitung und Rückführung des zum Waschen benutzten Ethanols, sowie ferner das erzeugte Futtermittel und Verwendungen für erzeugtes Glucosekonzentrat.

## Beschreibung

Die Erfindung betrifft ein Verfahren zur Herstellung eines wasserlöslichen Arabinoxylan aus Weizen sowie das hieraus gewonnene Futtermittel und die Verwendungen von erhaltenem Glucosekonzentrat, wobei eine Schließung des Wasserkreislaufes in Weizenstärkefabriken erreicht wird.

In Weizenstärkefabriken werden vorwiegend Weizenmehle von Weichweizen (*Triticum aestivum*) verarbeitet. Dabei werden die Hauptinhaltsstoffe Stärke und Weizenprotein in Separationsprozessen gewonnen. Nebenprodukte dieser Separation werden zu diversen Futtermitteln verarbeitet. Diese Futtermittel werden unter anderem aus den Nebenströmen, dem Prozesswasser, der Stärkefabrik hergestellt. Für die primären Separationsprozesse, also für die Trennung der Mehlkomponenten, werden liegende Zentrifugen, sogenannte Dekanter eingesetzt.

Im Stand der Technik wird zwischen Zwei-Phasen- und Drei-Phasen-Dekantern unterschieden, wobei das erfindungsgemäße Verfahren in beiden Fällen zum Einsatz gebracht werden kann und es nicht relevant ist, welche der beiden Maschinentypen eingesetzt werden. Die Dekanter haben die Aufgabe, die Weizeninhaltsstoffe in Abhängigkeit ihrer Sedimentationseigenschaften in unterschiedliche Fraktionen zu trennen.

Im Falle des Zwei-Phasen-Dekanters erfolgt die Trennung in eine Phase, die hauptsächlich Stärke enthält, und eine zweite Phase, die alle übrigen Mehlinhaltsstoffe enthält.

Der Drei-Phasein-Dekanter trennt die Inhaltsstoffe in eine hauptsächlich Stärke, in eine hauptsächlich Protein, und in eine dritte Phase, die alle löslichen Komponenten, somit auch der löslichen Ballaststoffe des Weizenmehls, enthält, und als Prozesswasser bezeichnet wird. Da diese Dreiteilung der Stoffströme die effektivste Stofftrennung darstellt, ist die Verwendung des Drei-Phasen-Dekanters im Verfahrensschritt des primären Stärkegewinnungsprozesses, als nächstliegender Stand der Technik zu betrachten. Ein solches Verfahren ist beispielsweise in der WO 2008/009733 A2 beschrieben worden.

Für die Verwertung dieses Prozesswassers mit den überwiegend löslichen Stoffkomponenten werden folgende Verfahren beschrieben und praktiziert:
- Konzentration der Trockensubstanz mit Verdampfern und Vertrieb des flüssigen Konzentrats als Tierfutter.
- Konzentration der Trockensubstanz mit Verdampfern und Aufsprühen auf Weizenkleie und Vertrieb als trockenes Tierfutter. Das Brüdenkondensat wird über den Vorfluter entsorgt.
- Verarbeitung im Biogasfermenter zur Energiegewinnung und Entsorgung der gereinigten Fermentationswässer im Vorfluter.
- Natürliche Sedimentation der Inhaltsstoffe in großen Sedimentationsbecken und Verregnung der neutralisierten, geklärten Wässer auf landwirtschaftlich genutzten Flächen und Verwertung der Sedimente als flüssige Tierfutter.
- Natürliche Fermentation der löslichen Mehlinhaltsstoffe bis zu einem pH-Wert von ungefähr 3,2 und Verkauf als fermentiertes Flüssigfutter (HAMINO®)

Nachteilig ist bei diesen Verfahren, dass entweder nur flüssiges Tierfutter erhältlich ist oder im Falle des Aufsprühens Weizenkleie als Grundstoff erforderlich ist. Außerdem fallen Abwässer in erheblichem Umfang an. Die Aufgabe der Erfindung ist es daher, ein abwasserfreies Verfahren zur Verfügung zu stellen, das alle Inhaltsstoffe verwerten und aus bislang schlecht verwertbaren Inhaltsstoffen einen wertvollen löslichen Ballaststoff, welcher Arabinoxylan als Hauptbestandteil enthält und ein proteinhaltiges, festes Tierfutter erzeugt.

Die Erfindung löst die Aufgabe, das abwasserfreie Verfahren zur Herstellung von wasserlöslichem Arabinoxylan aus Weichweizenmehl bereitzustellen, mittels der Verfahrensschritte:
a) Herstellung einer Mehl-Wasser-Suspension
b) Einhalten einer Ruhezeit der Mehl-Wasser-Suspension von 3-10 Minuten
c) Verdünnung der Mehl-Wasser-Suspension mit Kreislaufwasser,
d) Dekantieren der Mehl-Wasser-Suspension mittels eines Drei-Phasen-Dekanters in einem Zentrifugalfeld,
e) Separation des erhaltenen Oberlaufs in einem weiteren Zentrifugalfeld,
f) Rückführung der im weiteren Zentrifugalfeld erhaltenen schweren Fraktion in den Stärkegewinnungskreislauf,
g) Zusetzung von α-Amylase zur im weiteren Zentrifugalfeld erhaltenen leichten Fraktion,
h) Dextrinieren der leichten Fraktion mit einem Jet-Kocher,
i) Lagerung des Hydrolysats mit Flokkulation und Sedimentation in einem Reaktionsbehälter über 0,3 bis 13 Stunden und einem pH-Wert von 1 bis 10,
j) Abzentrifugieren des Sediments,
k) Mahltrocknung des Sediments,
l) Sterilisation des geklärten Hydrolysates
m) Verzuckern des sterilisierten, abgekühlten und dextrinierten Hydrolysates in einem Rührreaktor mittels Glucoamylase unter gleichzeitiger Proteolyse mit einer Mischung einer alkalischen und einer neutralen Protease,
n) Ultrafiltration des Hydrolysates über eine mehrstufige Ultrafiltration, wobei ein Permeat und ein Retentat erhalten werden und das Retentat mengenmäßig nur noch höchstens ein Fünftel der Ausgangsmenge beträgt,
o) Eindampfung des Permeats, bis eine Konzentration von 50 bis 90 % Trockensubstanz erreicht ist,
p) Eindampfung des Retentats, bis eine Konzentration von 20 bis 50 % Trockensubstanz erreicht ist,
q) Rückführung der beiden Verdampferbrüdenkondensate in den Stärkeprozess,
r) Präzipitation des eingedampften Retentats mittels konzentrierten Ethanols bis zu einer Ethanolkonzentration von 60 bis 80 Massenprozent in einem Kreislauf, der in Semi-Batch-Fahrweise betrieben wird und gebildet wird durch einen Rührreaktor enthaltend 94 bis 99 Volumenprozent Ethanol bei einer Temperatur von 5 bis 70 °C, weiterhin einen schnelllaufenden Rotor-Stator-Mischer, eine Zugabemöglichkeit von konzentriertem Retentat in einen Bypass-Einlass des Rotor-Stator-Mischers unter Vakuumbedingungen,
s) Filtern und mindestens einmaligem Waschen des Filterkuchens mit 94 bis 99 Volumenprozent aufweisendem Ethanol sowie anschließender Mahltrocknung, und
t) Aufarbeitung und Rückführung des zum Waschen benutzten Ethanols.

Die Verfahrensschritte a) bis d) verlaufen dabei analog dem herkömmlichen Stand der Technik. In Ausgestaltungen der Erfindung wird vorgesehen, dass
- der Gehalt an Trockensubstanz im Oberlauf von Schritt e) zwischen 6 und12 % eingestellt wird,
- der Gehalt an Trockensubstanz im Oberlauf von Schritt e) zwischen 8 und10 % eingestellt wird,
- zur im weiteren Zentrifugalfeld erhaltenen leichten Fraktion eine Schwefeldioxid abgebende Chemikalie zusätzlich zur α-Amylase zugesetzt wird,
- wobei die Schwefeldioxid abgebende Chemikalie Natriumbisulfit ist,
- oder wobei die Schwefeldioxid abgebende Chemikalie Kaliumdisulfit ist,
- die Dextrinierung in Schritt h) bei 80 bis 90 °C durchgeführt wird,
- als Verweilzeit im Reaktionsbhälter in Schritt i) 1 Stunde gewählt wird,
- als pH-Wert im Reaktionsbehälter in Schritt i) 4,5 bis 7,0 gewählt wird,
- als Trockner in der Mahltrocknung in Schritt k) ein Ultrarotor verwendet wird,
- die Verzuckerung in Schritt m) bei einer Reaktionstemperatur von 60 °C vorgenommen wird,
- für die Ultrafiltration in Schritt n) Membranen aus Polyethersulfon, welche eine Trenngrenze von 90.000 bis 110.000 Dalton, vorzugsweise 100.000 Dalton, aufweisen, verwendet werden,
- die Eindampfung des Permeats in Schritt o) durchgeführt wird, bis eine Konzentration von 70 %Trockensubstanz erreicht ist,
- der Gehalt an Glucose im eingedampften Permaeat in Schritt o) auf 50 bis 90 % der Trockensubstanz, vorzugsweise 70 bis 80 % der Trockensubstanz eingestellt wird,
- die Retentatkonzentration im Schritt p) auf 40 % der Trockensubstanz eingestellt wird,
- das im Rührreaktor in Schritt r) eingesetzte Ethanol mit einem Temperaturniveau von 15 bis 25 °C vorgelegt wird,
- konzentriertes Retentat im Bypass-Einlass des Rotor-Stator-Mischers in Schritt r) bei einer Temperatur von 5 bis 80 °C zugegeben wird,
- konzentriertes Retentat im Bypass-Einlass des Rotor-Stator-Mischers in Schritt r) bei einer Temperatur von 20 bis 40 °C zugegeben wird,
- die Präzipitation in Schritt r) bis zu einer Ethanolkonzentration von 6D bis 80 Massenprozent geführt wird,
- die Präzipitation in Schritt r) bis zu einer Ethanolkonzentration von 66 bis 68 Massenprozent geführt wird.

Die Erfindung löst dabei auch die Aufgabe, aus bislang schlecht verwertbaren Inhaltsstoffen ein wertvolles, festes Tierfutter zu erzeugen, indem durch ein Verfahren gemäß Anspruch 1 als mahlgetrockneter Filterkuchen im Schritt k) ein proteinhaltiges Futtermittel gewonnen wird. In optimierten Ausgestaltungen der Erfindung wird vorgesehen, dass der Proteingehalt des Futtermittels zwischen 15 und 50 %, vorzugsweise 35 % der Trockensubstanz beträgt. Während proteinhaltige Futtermittel, die bei der Ethanolherstellung aus Schlempe gewonnen werden, beispielsweise aus der DE 103 27 954 A1 bekannt sind, ist ein derartiges Futtermittel, welches nicht aus Schlempe gewonnen wird und daher in seiner Zusammensetzung hochwertiger ist, neu.

Weiterhin löst die Erfindung die Aufgabe, ein wasserlösliches Ballaststoffkonzentrat zu erzeugen, bei dem die Hauptkomponenten Arabinoxylan und Arabinogalactan sind. Hierbei besitzt das Endprodukt einen Gesamtballaststoffgehalt von 50 bis 80 % in der Trockensubstanz und hat einen Polymerisationsgrad von 100 bis 140 Kohlenhydratbausteinen. Balaststoffhaltige Mittel, die grundsätzlich auch Arabinoxylan und Arabinogalactan enthalten können, sind aufgrund ihrer cholesterinsenkenden Eigenschaften beispielsweise aus der DE 102 33 342 A1 bekannt, das hier gewonnene ballaststoffhaltige Mittel ist jedoch außerdem von Natur aus wasserlöslich. Es ist ferner frei von Ethanol.

Vorteile der Erfindung sind auch die Verwendbarkeit des gewonnenen Glucose-konzentrats aus dem eingedampften Permeat des Verfahrensschritts o) in der Fermentationsindustrie, zur Herstellung von Backhefen und als Presshilfsmittel in der Futtermittel-Pelletproduktion.

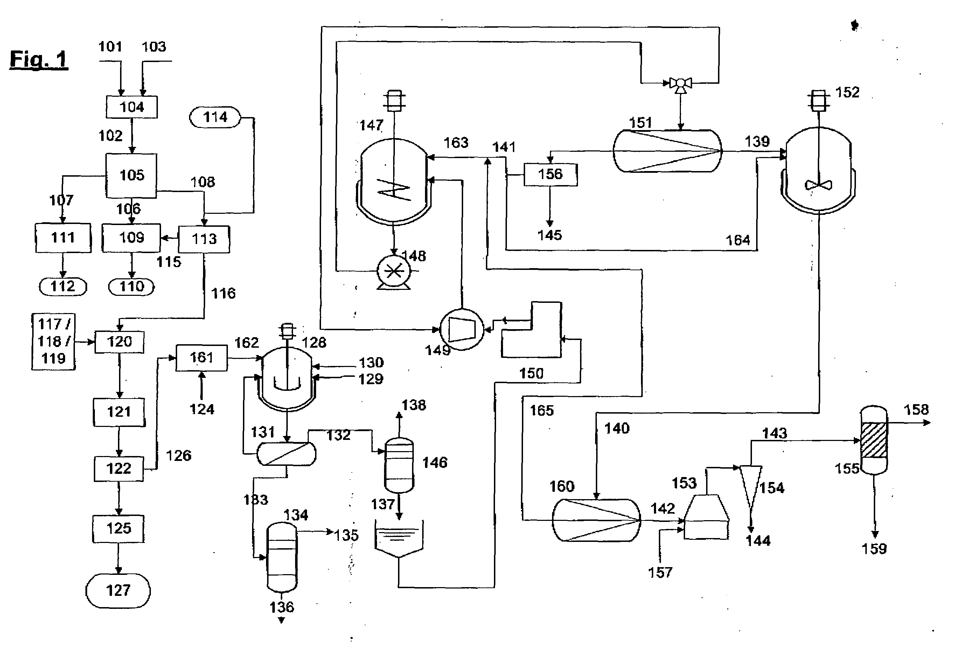

Die Erfindung wird im Folgenden anhand eines vereinfachten Verfahrensfließbildes in Fig. 1 näher erläutert.

Der Trennprozess des Mehles erfolgt zunächst analog dem herkömmlichen Stand der Technik. Nach der Herstellung einer Mehl-Wasser-Dispersion 104 aus Kreislaufwasser 101 und Stärkemehl 103 wird diese einer Teigruhe von ca. 3-10 min überlassen. Nach dieser Teigruhe wird die Suspension mit Kreislaufwasser 101 auf eine, für die Trennung im Zentrifugalfeld eines Dekanters 105 geeignete Konzentration verdünnt und im Zentrifugalfeld eines Drei-Phasen-Dekanters getrennt.

Dabei wird die Hauptmenge an A-Stärke 106 in die schwere Phase, dem Unterlauf, separiert. Das agglomerierte, unlösliche Protein 107 wird zusammen mit Stärke und löslichen Inhaltsstoffen in der mittleren Phase abgeschieden und zur Glutenraffination 111 geführt, wo Gluten 112 hergestellt wird. Die mittlere Phase wird zur Stärkeraffination 109 geleitet, wo A-Stärke 110 hergestellt wird. Als leichteste Phase, der Oberlauf 108, wird der Hauptteil der löslichen Komponenten, somit auch der löslichen Ballaststoffe des Weizenmehls aus dem System abgetrennt.

Dieser Öberlauf 108 hat einen Trockensubstanzgehalt von 6 bis 16 %. In dieser Phase befinden sich nicht-agglomerierte Proteine, Feinpartikelstärke, Bruchstücke von Stärkekömem, Feinstfasern und die löslichen Bestandteile wie das Arabinoxylan, Pentosane und Hemicellulosen. Hier setzt das erfindungsgemäße Verfahren zur Gewinnung eines wasserlöslichen Arabinoxylan ein, welches auch parallel zur Erzeugung von Flüssigfuttermittel Hamino 114 erfolgen kann.

Der Oberlauf 108 wird zur Entfernung des Großteils Stärke und agglomerierbarem Proteins sofort und ohne Zwischenlagerung erneut einer Separation 113 in einem Zentrifugalfeld unterzogen. Die schwere Fraktion 115 dieser Separation 113 gelangt in den Stärkegewinnungskreislauf zurück. Die leichte Fraktion (Prozesswasser) 116 wird zur Verarbeitung im erfindungsgemäßen Verfahren verwendet.

Der gereinigte stärkehaltige Oberlauf (Prozesswasser) 116 hat einen Gehalt an Trockensubstanz von 6 bis 12 % vorzugsweise von 8 bis 10 %, wird mit Dampf 117 und einer handelsüblichen α-Amylase 118 und wahlweise mit einer Schwefeldioxid abgebenden Chemikalie 119, wie beispielsweise Natriumbisulfit, vorzugsweise Kaliumdisulfit (K₂S₂O₅), versetzt und mit einem Jet-Kocher 120 bei der für das verwendete Enzym optimalen Reaktionstemperatur, vorzugsweise bei 80 bis 90 °C, kontinuierlich dextriniert. Dieses Hydrolysat wird für 0,30 bis 13,0 h, vorzugsweise 1 h, bei einem pH-Wert von 1,0 bis 10,0 vorzugsweise bei 4,5 bis 7,0 in einem Reaktionsbehälter 121 gelagert.

Während dieser Lagerung kommt es zu einer Flokkulation der bis dahin löslichen Proteine und Liquide. Der sedimentierbare Anteil wird im Zentrifugalfeld 122 von Separatoren oder Dekantern abgetrennt und zu einem neuartigen Futtermittel 127, mit einem Proteingehalt zwischen 15 bis 50 %, vorzugsweise 35 % der Trockensubstanz verarbeitet. Dazu wird das Sediment 123 mit einem geeigneten Trockner 125, vorzugsweise mit einem Ultrarotor, in einem Mahl-Trocknungs-Prozess getrocknet.

Das dextrinierte, geklärte Hydrolysat 126 wird in einem Jetkocher 161 unter Zusatz von Dampf 124 sterilisiert, das sterilisierte Hydrolysat 162 wird danach in einen Rührreaktor 128 gegeben. Hierin verzuckert das Hydolysat bei der optimalen Reaktionstemperatur, vorzugsweise 60 °C für das für die Verzuckerung der löslichen Glucane üblicherweise verwendete Glucoamylasepräparat 129. Gleichzeitig wird es mit einer Mischung einer alkalischen und einer neutralen Protease 130 einer Proteolyse unterzogen.

Während dieser enzymatischen Behandlung wird das Hydrolysat über eine mehrstufige Ultrafiltration 131 ultrafiltriert. Vorzugsweise kommen Membranen aus Polyethersulfon, welche eine Trenngrenze von ungefähr 100.000 Dalton aufweisen, zum Einsatz. Mit dieser Membran werden die Ballaststoffe effektiv zurückgehalten und im Retentat 132 konzentriert. Die Ultrafiltration wird bis auf ein Fünftel der Ausgangsmenge geführt.

Das Permeat 133 enthält den Großteil der löslichen Proteine und der gebildeten Glucose und wird mit einem Verdampfer 134 geeigneter Konstruktion auf einen Gehalt an Trockensubstanz (im folgenden mit TS bezeichnet) von 50 bis 90 %, vorzugsweise 70 %, konzentriert. Dieses Glucosekonzentrat 136 mit einem Gehalt an Glucose von 50 bis 90 % der TS, vorzugsweise 70 bis 80 % der TS, kann vorteilhaft in der Fermentationsindustrie, zum Beispiel, zur Herstellung von Backhefen oder auch als Presshilfsmittel in der Futtermittel-Pelletproduktion eingesetzt werden. Das Verdampfer-Kondensat 135 wird erfindungsgemäß in den Stärkeprozess zurückgeführt.

Erfindungsgemäß wird das Retentat 132 wie folgt weiterverarbeitet. Das Retentat 132 wird in einem Verdampfer 146 auf einen TS-Gehalt von 20 bis 50 % (137), vorzugsweise 40 % TS, konzentriert. Das Brüdenkondensat 138 wird erfindungsgemäß wieder in der Stärkefabrikation eingesetzt. Das Retentatkonzentrat 137 wird zur erfindungsgemäßen Herstellung eines mit Arabinoxylan angereicherten Ballaststoffkonzentrats verwendet.

Zur Herstellung des erfindungsgemäßen Ballaststoffkonzentrates wird in einem Rührreaktor 147 94 bis 99 Volumenprozent Ethanol 141, welches ein Temperaturniveau von 5 bis 70 °C, vorzugsweise von 15 bis 25 °C besitzt, vorgelegt. Das Ethanol wird mittels einer Verdrängerpumpe 148 in einen schnelllaufenden Rotor-Stator-Mischer 149 im Kreislauf gepumpt. Durch das Spaltsystem im Rotor-Stator-Mischer 149 wird an einem, am Mischer angebrachten Bypass-Einlass ein Vakuum erzeugt. In dieses Vakuum wird nun, unter Zuhilfenahme einer Verdrängerpumpe 150, das konzentrierte Retentat 137, welches eine Temperatur von 5 bis 80 °C, vorzugsweise 20 bis 40 °C hat, gepumpt. Im Spaltsystem des Rotor-Stator-Mischers 149 wird das konzentrierte Retentat 137 mit dem Ethanol gemischt und feinst verteilt. Der Ballaststoff wird durch den Kontakt mit dem konzentrierten Ethanol sofort präzipitiert.

Die Inhaltsstoffe, welche nicht im Ethanol präzipitieren, verbleiben gelöst im Ethanol. Die Präzipitation wird bis zu einer Ethanolkonzentration von 60 bis 80 Massenprozent, vorzugsweise bis zu einer Konzentration von 66 bis 68 Massenprozent Ethanol geführt. Danach wird die Präzipitation abgebrochen und der Inhalt des Rührreaktors 147 abgepumpt und mittels einer Filterpresse 151 abgefiltert. Der entstehende Filterkuchen 139 wird abgepresst. Der ethanolfeuchte Filterkuchen 139 wird nun erneut mit 94 bis 99 Volumenprozent Ethanol 164 gewaschen, dieser Schritt findet im Rührbehälter 152 statt. Dabei wird das Ballaststoffkonzentrat von anhaftenden, löslichen Begleitstoffen weitgehend freigewaschen. Das den Ballaststoff enthaltende Ethanol 140 wird wiederum mittels einer Filterpresse 160 ent feuchtet, das abgeschiedene konzentrierte Ethanol 165 wird mit konzentriertem Ethanol 141 aus der Ethanolaufbereitung 156 gemischt, das sich ergebende konzentrierte Ethanol 163 wird im Rührbehälter 147 eingesetzt.

Das zum Waschen des Filterkuchens benutzte Ethanol wird erneut in den Fällungsprozess geführt. Der ethanolfeuchte und gewaschene Filterkuchen 142, ein Ballaststoffkonzentrat, wird in einem Ultrarotor-System 153 mahlgetrocknet, wobei Luft 157 zugeführt wird. Das getrocknete und gemahlene Ballaststoffkonzentrat 144 wird über Filtersysteme 154 abgeschieden und kann nun verpackt werden.

Das erhaltene Ballaststoffkonzentrat 144 ist ein wasserlösliches Ballaststoffkonzentrat, bei dem die Hauptkomponenten Arabinoxylan und Arabinogalactan sind. Hierbei besitzt das Endprodukt einen Gehalt an Arabinoxylan von 50 bis 80 % in der Trockensubstanz und hat ein durchschnittliches Molekulargewicht im Bereich von 140.000 g/mol und einen Polymerisationsgrad von 100 bis 140 Kohlenhydratbausteinen.

Das Ultra-Rotor-System hat ein integriertes Lösungmittelrückgewinnungssystem 155. Das rückgewonnene Ethanol wird zusammen mit dem Ethanol aus der Präzipitation in der Ethanolaufbereitung wieder aufgearbeitet 156 und erneut dem Prozess der Präzipitation zugeführt. Das Konzentrat 145 aus der Ethanolaufarbeitung 156 wird dem Glucosekonzentrat 136 zugeführt. Die Abluft 158, die das Lösungsmittelrückgewinnungssystem verlässt, ist Ethanol-arm, das zurückgewonnene Ethanol 159 kann wiederverwendet werden.

Auf die in der erfindungsgemäßen beschriebenen Art und Weise können die Produktions-Waschwässer einer Weizenstärkefabrik gewinnbringend verarbeitet und vollkommen verwertet werden. Es gibt keine Abwässer, welche entsorgt werden müssen.

Zur Veranschaulichung der Funktionsweise dient das folgende Auslegungsbeispiel einer Anlage nach dem erfindungsgemäßen Verfahren mit den wichtigsten Stoffströmen und Betriebsmitteln.

| Nr. | Massenstrom | Trockensubstanz | | Wasser | Ethanol |
|---|---|---|---|---|---|
| | kg/h | kg/h | % TS | kg/h | kg/h |
| 108 | 20000 | 2190 | 10,95 | 17810 | - |
| 116 | 16980 | 1344 | 7.9 | 15636 | - |
| 120 | 18156 | 1344 | 7,4 | 16812 | - |
| 120 | 19133 | 1344 | 7,0 | 17789 | - |
| 121 | 14973 | 849 | 5,7 | 14124 | - |
| 126 | 4160 | 495 | 11,9 | 3665 | - |
| 125 | 12136 | 607 | 5,0 | 11529 | - |
| 127 | 2837 | 218 | 7,7 | 2618 | - |
| 127 | 1242 | 621 | 50 | 621 | - |
| 137 | 945 | 208 | 22 | 737 | - |
| 141 | - | - | - | - | 1950 |
| 139 | 250 | 100 | 40 | - | 150 |
| 140 | 500 100 | - | - | - | 400 |
| 142 | 293 | 94 | 32 | - | 199 |
| 145 | 192 | 96 | 50 | 96 | - |
| 159 - | - | - | - | - | 290 |
| 165 | 207 | 6 | - | 201 | - |
| 144 | 97 | 94 | - | - | 3 |

Bezugszeichenliste
- 101: Kreislaufwasser 1
- 102: Kreislaufwasser 2
- 103: Stärkemehl
- 104: Mehl-Wasser-Mischer
- 105: 3-Phasen-Trenndekanhter
- 106: 1. Phase A-Stärke
- 107: 2. Phase Protein
- 108: 3. Phase Oberlauf
- 109: Stärkeraffination
- 110: A-Stärke
- 111: Glutenraffination
- 112: Gluten
- 113: Separation
- 114: Flüssigfuttermittel Hamino
- 115: Separator-Unterlauf
- 116: Separator-Oberlauf
- 117: Dampf für Jetkocher
- 118: α-Amylase
- 119: SO²
- 120: Jet-Kocher
- 121: Reaktionsbehälter
- 122: Separation
- 123: Separator-Unterlauf
- 124: Dampf
- 125: Trocknung
- 126: Dextriniertes, geklärtes Hydrolysat
- 127: Proteinhaltiges Futtermittel
- 128: Reaktionsbehälter
- 129: Gluco-Amylase
- 130: Protease
- 131: Ultrafiltration
- 132: Retentat
- 133: Permeat
- 134: Verdampfer
- 135: Brüdenkondensat
- 136: Glucosekonzentrat
- 137: Retentatkonzentrat
- 138: Brüdenkondensat
- 139: AX-haltiger Filterkuchen
- 140: Filtriertes Ethanol
- 141: Konzentriertes Ethanol
- 142: Gewaschenes Balaststoffkonzentrat
- 143: Ethanolhaltige Abluft
- 144: Getrocknetes, balaststoffreiches AX-angereichertes Balaststoffkonzentrat
- 145: Glucosekonzentrat
- 146: Verdampfer
- 147: Rührbehälter
- 148: Pumpe
- 149: Rotor-Stator-Mischer
- 150: Dosierpumpe
- 151: Filterpresse 1
- 152: Rührbehälter
- 153: Ultra-Rotor
- 154: Abscheider
- 155: Ethanol-Rückgewinnungssystem
- 156: Ethanolaufarbeitung
- 157: Luft
- 158: Abluft
- 159: Ethanol
- 160: Filterpresse 2
- 161: Jetkocher
- 162: Sterilisiertes Hydrolysat
- 163: Konzentriertes Ethanol
- 164: Konzentriertes Ethanol
- 165: Konzentriertes Ethanol

## Patentansprüche

1. Verfahren zur Herstellung von wasserlöslichem Arabinoxylan aus Weichweizenmehl mittels der Verfahrensschritte
a) Herstellung einer Mehl-Wasser-Suspension (104)
b) Einhalten einer Ruhezeit der Mehl-Wasser-Suspension von 3-10 Minuten
c) Verdünnung der Mehl-Wasser-Suspension mit Kreislaufwasser,
d) Dekantieren der Mehl-Wasser-Suspension (102) mittels eines Drei-Phasen-Dekanters (105) in einem Zentrifugalfeld,
e) Separation des erhaltenen Oberlaufs (108) in einem weiteren Zentrifugalfeld,
f) Rückführung der im weiteren Zentrifugalfeld erhaltenen schweren Fraktion (106) in den Stärkegewinnungskreislauf,
g) Zusetzung von α-Amylase (118) zur im weiteren Zentrifugalfeld erhaltenen leichten Fraktion,
h) Dextrinieren der leichten Fraktion mit einem Jet-Kocher (120),
i) Lagerung des Hydrolysats mit Flokkulation und Sedimentation in einem Reaktionsbehälter (121) über 0,3 bis 13 Stunden und einem pH-Werk von 1 bis 10,
j) Abzentrifugieren des Sediments,
k) Mahltrocknung des Sediments,
l) Sterilisation des geklärten Hydrolysates
m) Verzuckern des sterilisierten, abgekühlten und dextrinierten Hydrolysates in einem Rührreaktor (128) mittels Glucoamylase (129) unter gleichzeitiger Proteolyse mit einer Mischung einer alkalischen und einer neutralen Protease (130),
n) Ultrafiltration des Hydrolysates über eine mehrstufige Ultrafiltration, wobei ein Permeat (133) und ein Retentat (132) erhalten werden und das Retentat mengenmäßig nur noch höchstens ein Fünftel der Ausgangsmenge beträgt,
o) Eindampfung des Permeats (133), bis eine Konzentration von 50 bis 90 % Trockensubstanz erreicht ist,
p) Eindampfung des Retentats (132), bis eine Konzentration von 20 bis 50 % Trockensubstanz erreicht ist,
q) Rückführung der beiden Verdampferbrüdenkondensate in den Stärkeprozess,
r) Präzipitation des eingedampften Retentats (137) bis zu einer Ethanolkonzentration von 60 bis 80 Massenprozent in einem Kreislauf, der in Semi-Batch-Fahrweise betrieben wird und gebildet wird durch einen Rührreaktor (147), enthaltend 94 bis 99 Volumenprozent Ethanol bei einer Temperatur von 5 bis 70 °C, weiterhin einen schnelllaufenden Rotor-Stator-Mischer (149), eine Zugabemöglichkeit von konzentriertem Retentat in einen Bypass-Einlass des Rotor-Stator-Mischers unter Vakuumbedingungen,
s) Filtern und mindestens einmaligem Waschen des Filterkuchens mit 94 bis 99 Volumenprozent aufweisendem Ethanol sowie anschließender Mahltrocknung (153), und
t) Aufarbeitung und Rückführung des zum Waschen benutzten Ethanols.

2. Verfahren nach Anspruch 1, **dadurch, gekennzeichnet, dass** der Gehalt an Trockensubstanz im Oberlauf von Schritt e) zwischen 6 und12 %, vorzugsweise zwischen 8 und 10 %, eingestellt wird.

3. Verfahren nach Anspruch 1, **dadurch gekennzeichnet, dass** zur im weiteren Zentrifugalfeld erhaltenen leichten Fraktion eine Schwefeldioxid abgebende Chemikalie, vorzugsweise ausgewählt aus der Gruppe, die gebildet wird aus Natriumbisulfit und Kaliumdisulfit, zusätzlich zur α-Amylase zugesetzt wird.

4. Verfahren nach Anspruch 1, **dadurch gekennzeichnet, dass** die Dextrinierung in Schritt h) bei 80-90 °C durchgeführt wird,
- als Verweilzeit im Reaktionsbehälter in Schritt i) 1 Stunde gewählt wird,
- als pH-Wert im Reaktionsbehälter in Schritt i) 4,5 bis 7,0 gewählt wird,
- als Trockner in der Mahltrocknung in Schritt k) ein Ultrarotor verwendet wird,
- die Verzuckerung in Schritt m) bei einer Reaktionstemperatur von 60 °C vorgenommen wird,
- für die Ultrafiltration in Schritt n) Membranen aus Polyethersulfon, welche eine Trenngrenze von 90.000 bis 110.000 Dalton, vorzugsweise 100.000 Dalton, aufweisen, verwendet werden,
- die Eindampfung des Permeats in Schritt o) durchgeführt wird, bis eine Konzentration von 70 %Trockensubstanz erreicht ist.

5. Verfahren nach Anspruch 1, **dadurch gekennzeichnet, dass** die Eindampfung des Permeats (133) in Schritt o) durchgeführt wird, bis eine Konzentration von 70 %Trockensubstanz erreicht ist, und dass der Gehalt an Glucose im eingedampften Permaeat (136) in Schritt o) auf 50 bis 90 % der Trockensubstanz, vorzugsweise 70 bis 80 % der Trockensubstanz eingestellt wird.

6. Verfahren nach Anspruch 1, **dadurch gekennzeichnet, dass** die Retentatkonzentration im Schritt p) auf 40 % der Trockensubstanz eingestellt wird.

7. Verfahren nach Anspruch 1, **dadurch gekennzeichnet, dass**
- das im Rührreaktor (147) in Schritt r) eingesetzte Ethanol mit einem Temperaturniveau von 15 bis 25 °C vorgelegt wird,
- konzentriertes Retentat im Bypass-Einlass des Rotor-Stator-Mischers (149) in Schritt r) bei einer Temperatur von 5 bis 80 °C, vorzugsweise bei einer Temperatur von 20 bis 40 °C, zugegeben wird, und
- die Präzipitation in Schritt r) bis zu einer Ethanolkonzentration von 60 bis 80 Massenprozent, vorzugsweise bis zu einer Ethanolkonzentration von 66 bis 68 Massenprozent, geführt wird.

8. Verwendung des in einem Verfahren entsprechend Anspruch 5 erhaltenen Glucosekonzentrats (136) in der Fermentationsindustrie.

9. Verwendung des in einem Verfahren entsprechend Anspruch 5 erhaltenen Glucosekonzentrats (136) zur Herstellung von Backhefen.

10. Verwendung des in einem Verfahren entsprechend Anspruch 5 erhattenen Glucose-konzentrats (136) als Presshilfsmittel in der Futtermittel-Pelletproduktion

11. Futtermittel (127), enthaltend Protein, erhalten durch ein Verfahren gemäß einem der Ansprüche 1 bis 7, als mahlgetrockneter Filterkuchen im Schritt k), **dadurch gekennzeichnet, dass** der Proteingehalt zwischen 15 und 50 %, vorzugsweise 35 % der Trockensubstanz beträgt.

12. Wasserlösliches Ballaststoffkonzentrat (144), **dadurch gekennzeichnet, dass** die Hauptkomponenten Arabinoxylan und Arabinogalactan sind.

13. Wasserlösliches Ballaststoffkonzentrat (144), **dadurch gekennzeichnet dass** das Endprodukt einen Gesamtballaststoffgehalt von 50 bis 80 % in der Trockensubstanz besitzt.

14. Wasserlösliches Ballaststoffkonzentrat (144), **dadurch gekennzeichnet, dass** es einen Polymerisationsgrad von 100 bis 140 Kohlenhydratbausteinen hat.
